# EUROPÄISCHE PATENTSCHRIFT

(11) **EP 2 193 128 B1**
(45) Veröffentlichungstag und Bekanntmachung des Hinweises auf die Patenterteilung: **26.10.2016**
(21) Anmeldenummer: 08801865.0
(22) Anmeldetag: 05.09.2008
(51) Int. Cl.: C07D 307/68

(54) **VERFAHREN ZUR HERSTELLUNG VON 4-AMINOBUT-2-ENOLIDEN**
METHOD FOR MANUFACTURING 4-AMINOBUT-2-ENOLIDS
PROCÉDÉ DE FABRICATION DE 4-AMINOBUT-2-ÉNOÏQUE

(30) Priorität: 18.09.2007 EP 07116639
(43) Veröffentlichungstag der Anmeldung: 09.06.2010
(73) Patentinhaber: Bayer Intellectual Property GmbH, 40789 Monheim am Rhein (DE)
(72) Erfinder: LUI, Norbert, 51519 Odenthal (DE); HEINRICH, Jens-Dietmar, 51399 Burscheid (DE)
(74) Vertreter: BIP Patents
(86) Internationale Anmeldenummer: PCT/EP2008/007270
(87) Internationale Veröffentlichungsnummer: WO 2009/036899

(56) Entgegenhaltungen:
- EP-A- 0 539 588
- MITSOS, CHRISTOS ET AL: "Synthesis of tetronic acid derivatives from novel active esters of .alpha.-hydroxyacids" JOURNAL OF HETEROCYCLIC CHEMISTRY , 39(6), 1201-1205 CODEN: JHTCAD; ISSN: 0022-152X, 2002, XP002473738
- ATHANASELLIS, GIORGOS ET AL: "Novel short-step synthesis of optically active tetronic acids from chiral .alpha.-hydroxy acids mediated by 1-hydroxybenzotriazole" SYNLETT , (10), 1736-1738 CODEN: SYNLES; ISSN: 0936-5214, 2002, XP002473739
- CAMPBELL A C ET AL: "SYNTHESIS OF (E)- AND (Z)-PULVINONES" JOURNAL OF THE CHEMICAL SOCIETY, PERKIN TRANSACTIONS 1, CHEMICAL SOCIETY. LETCHWORTH, GB, Bd. 1, 1985, Seiten 1567-1576, XP001005573 ISSN: 0300-922X in der Anmeldung erwähnt

## Beschreibung

Die vorliegende Erfindung betrifft ein Verfahren zur Herstellung von 4-Amino-but-2-enoliden sowie von entsprechenden Intermediaten bzw. Ausgangsverbindungen, welche in dem erfindungsgemäßen Verfahren durchlaufen bzw. verwendet werden.

Bestimmte substituierte 4-Aminobut-2-enolid-Verbindungen sind als insektizid wirksame Verbindungen aus der EP 0 539 588 A1 bekannt. Darüber hinaus beschreiben auch die internationalen Patentanmeldungen WO 2007/115644, WO 2007/115643 und WO 2007/115646 entsprechende insektizid wirksame 4-Aminobut-2-enolid-Verbindungen.

Im Allgemeinen werden Enaminocarbonylverbindungen aus Tetronsäure und einem Amin gemäß dem nachfolgenden Schema 1 synthetisiert. Diese Vorgehensweise ist beispielsweise in EP 0 539 588 A1 sowie in Heterocycles Vol. 27, Nr. 8, Seite 1907 bis 1923 (1988) beschrieben.

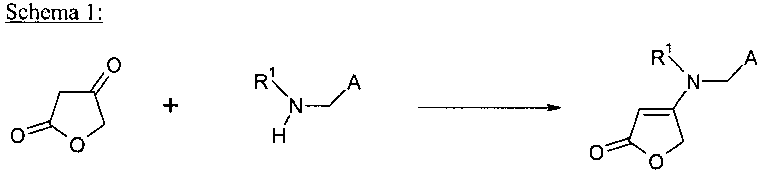

Nachteilig an diesem Verfahren ist insbesondere, dass man wasserfreie Tetronsäure als Ausgangsverbindung benötigt, deren Herstellung aufwendig und kostenintensiv ist.

So wird Tetronsäure im Allgemeinen ausgehend von Acetessigester über eine Bromierung und anschließende Hydrierung hergestellt (vgl. Synthetic Communication, 11 (5), Seiten 385 bis 390 (1981)). Die Gesamtausbeute an Tetronsäure ausgehend von Acetessigester ist dabei kleiner als 40 %, was das Verfahren unter industriellem Gesichtspunkt wenig attraktiv macht.

In der CH-PS 503 722 ist ein weiteres Verfahren zur Herstellung von Tetronsäure beschrieben. Dabei wird 4-Chloracetessigester mit einem aromatischen Amin zum 3-Arylaminocrotonlacton umgesetzt und anschließend wird die Tetronsäure durch Behandlung mit Mineralsäuren freigesetzt. Der Nachteil an diesem Verfahren besteht darin, dass die Isolierung der Tetronsäure nur durch Hochvakuum-Sublimation möglich ist, was auch dieses Verfahren unter industriellem Gesichtspunkt wenig attraktiv macht.

Ein weiteres Verfahren zur Herstellung von Tetronsäure ist in der EP 0 153 615 A beschrieben, in welchem von 2,4-Dichloracetessigestem ausgegangen wird. Dieses ebenfalls mehrstufige und aufwändige Verfahren liefert die gewünschte Verbindung ebenfalls nur mit einer mäßigen Gesamtausbeute von 65 %.

In Tetrahedron Letters, Nr. 31, Seiten 2683 und 2684 (1974) ist die Herstellung von Tetronsäure und einer entsprechenden Enaminocarbonylverbindung beschrieben. Die dort beschriebene Synthese ist in folgendem Schema 2 wiedergegeben. Als Edukt wird dabei Acetylendicarbonsäuredimethylester eingesetzt.

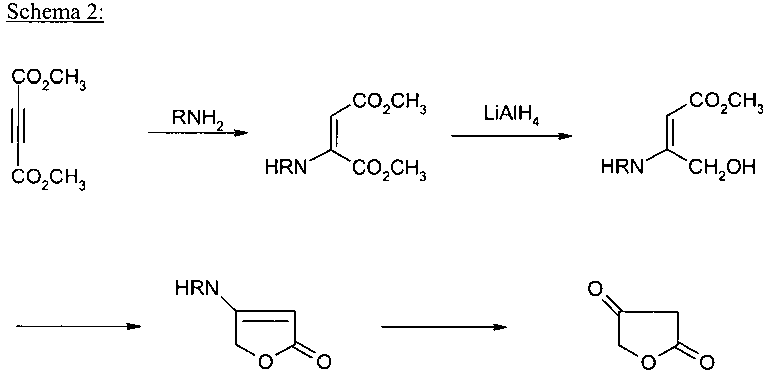

Nachteilig an diesem Verfahren ist die geringe Gesamtausbeute von nur 30 % sowie das Erfordernis, kostenintensive Edukte, beispielsweise Lithiumaluminiumhydrid (LiAlH₄), als Reagenzien verwenden zu müssen.

Ferner ist aus dem Stand der Technik ein Verfahren zur Herstellung von 4-Aminobut-2-enoliden ausgehend von Methyltetronat bekannt (J. Heterocyclic Chem., 21, 1753 (1984)). Für dieses Verfahren wird als Ausgangsmaterial der kostenintensive 4-Brom-3-methoxy-but-3-encarbonsäureester verwendet.

Ein weiteres Verfahren geht von einem 4-Chloracetessigester aus, der mit Aminen umgesetzt wird (Heterocycles, Vol. 27, Nr. 8, 1988, Seiten 1907 bis 1923). Die Reaktion zum Aminofuran wird in einem Schritt durchgeführt. Dabei wird das Amin mit Eisessig zu einer Lösung von 4-Chloracetessigester in Benzol gegeben und die resultierende Mischung wird mehrere Stunden unter Rückfluss erhitzt. Die Ausbeuten an 4-Methylamino-2(5H)-furanon in dieser Synthese betragen nur 40 %.

Aus EP 0 123 095 A ist ein Verfahren bekannt, in welchem Tetronsäureamid aus 3-Amino-4-acetoxycrotonsäureester hergestellt wird. 3-Amino-4-acetoxycrotonsäureester ist kostenintensiv und aufwändig herzustellen, so dass eine wirtschaftliche Synthese mit diesem Verfahren nicht möglich ist.

Ein weiteres Verfahren zur Herstellung von Tetronsäure ausgehend von Malonestern und Chloracetylchlorid ist aus J. Chem. Soc., Perkin Trans. 1 (1972), Nr. 9/10, Seiten 1225 bis 1231 bekannt. Dieses Verfahren liefert die gewünschte Zielverbindung mit einer Ausbeute von nur 43 %.

In der vorstehend erwähnten, Internationalen Patentanmeldung WO 2007/115644 wird die Herstellung von 4-Aminobut-2-enoliden, beispielsweise von 4-[[(6-Chlorpyridin-3-yl)methyl](3,3-dichlorprop-2-en-1-yl)amino]furan-2(5H)-on durch Umsetzung von 4-[[(6-Chlorpyridin-3-yl)methyl]amino]furan-2(5H)-on mit 3-Brom-1,1-dichlorprop-1-en, beschrieben (vgl. Herstellungsbeispiel, Verfahren 2, Beispiel (3)). Die WO 2007/115644 beschreibt auch die Herstellung von 4-Aminobut-2-enoliden, beispielsweise von 4-[[(6-Chlorpyridin-3-yl)methyl](3,3-dichlorprop-2-en-1-yl)amino]furan-2(5H)-on durch Umsetzung von 4-[[(2-Fluorethyl)amino]furan-2(5H)-on mit 2-Chlor-5-chlormethyl-pyridin (vgl. Herstellungsbeispiele, Verfahren 3, Beispiel (4)). Die Reaktionen werden vorzugsweise mit Hydriden des Lithiums oder Natriums durchgeführt. Diese Substrate sind im Allgemein kostenintensiv und gleichzeitig aus Sicherheitsgründen nur schwer zu handhaben.

Ausgehend von diesem Stand der Technik ergibt sich als Aufgabe der vorliegenden Erfindung, ein Verfahren zur Herstellung von 4-Aminobut-2-enolid-Verbindungen bereitzustellen, welches vorzugsweise einfach und kostengünstig durchzuführen ist. Die mit diesem angestrebten Verfahren erhältlichen 4-Aminobut-2-enolid-Verbindungen sollen dabei vorzugsweise mit hoher Ausbeute und hoher Reinheit erhalten werden. Insbesondere soll das angestrebte Verfahren den Erhalt der gewünschten Zielverbindungen ohne die Notwendigkeit komplexer Aufreinigungsmethoden ermöglichen.

Gelöst wird diese Aufgabe durch ein Verfahren zur Herstellung von 4-Aminobut-2-enolid-Verbindungen der allgemeinen Formel (I):

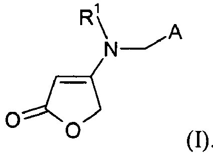

Das erfindungsgemäße Verfahren ist dadurch gekennzeichnet, dass man Verbindungen der allgemeinen Formel (II) 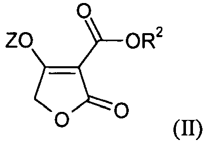 mit Aminen der allgemeinen Formel (III) 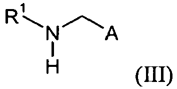 umsetzt, wobei die einzelnen Reste folgende Bedeutung aufweisen:
- R¹: Wasserstoff, Alkyl, Halogenalkyl, Alkenyl, Halogenalkenyl, Alkinyl, Cycloalkyl, Cycloalkylalkyl, Halogencycloalkyl, Alkoxy, Alkyoxyalkyl, Halogencycloalkylalkyl oder Arylalkyl;
- R²: Alkyl, Aryl oder Arylalkyl;
- Z: Wasserstoff, Alkalimetall oder Erdalkalimetall;
- A: Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder 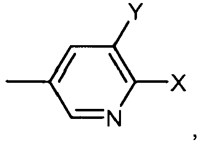 in welchem
- X: für Halogen, Alkyl oder Halogenalkyl steht und
- Y: für Halogen, Alkyl, Halogenalkyl, Halogenalkoxy, Azido oder Cyan steht.

Erfindungsgemäß ist somit vorgesehen, dass die gewünschten 4-Aminobut-2-enolid-Verbindungen der allgemeinen Formel (I) durch eine Umsetzung der entsprechenden Tetronsäureester der allgemeinen Formel (II) mit Aminen der allgemeinen Formel (III) hergestellt werden. Die gewünschten 4-Aminobut-2-enolid-Verbindungen der allgemeinen Formel (I) werden unter den erfindungsgemäßen und weiter unten näher spezifizierten bevorzugten Reaktionsbedingungen mit guten Ausbeuten in hoher Reinheit erhalten, womit das erfindungsgemäße Verfahren die oben genannten Nachteile der Verfahren des Standes der Technik überwindet. Die gewünschten Verbindungen werden dabei in einer Reinheit erhalten, welche eine umfangreiche Aufarbeitung des unmittelbaren Reaktionsprodukts im Allgemeinen nicht erforderlich macht.

Bevorzugte, besonders bevorzugte und ganz besonders bevorzugte Bedeutungen des in den oben erwähnten allgemeinen Formeln (I) und (III) aufgeführten Restes A werden im Folgenden erläutert.
- A: wird bevorzugt ausgewählt aus der Gruppe, bestehend aus 6-Fluor-pyrid-3-yl, 6-Chlor-pyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlor-pyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl und 5-Difluormethyl-6-iod-pyrid-3-yl.
- A: wird besonders bevorzugt ausgewählt aus der Gruppe, bestehend aus 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl und 5-Difluormethyl-6-chlor-pyrid-3-yl.
- A: wird ganz besonders bevorzugt ausgewählt aus der Gruppe, bestehend aus 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl.

Bevorzugte, besonders bevorzugte und ganz besonders bevorzugte Bedeutungen der in der oben erwähnten allgemeinen Formel (II) aufgeführten Reste Z, R¹ und R² werden im Folgenden erläutert.
- Z: wird bevorzugt ausgewählt aus der Gruppe, bestehend aus Alkalimetallen und Wasserstoff;
- Z: wird besonders bevorzugt ausgewählt aus der Gruppe, bestehend aus Wasserstoff, Natrium und Kalium;
- Z: ist ganz besonders bevorzugt Natrium oder Wasserstoff;
- R¹: wird bevorzugt ausgewählt aus Wasserstoff, Alkyl, Halogenalkyl, Alkenyl, Halogenalkenyl, Alkinyl, Cycloalkyl, Cycloalkylalkyl, Halogencycloalkyl, Halogencycloalkylalkyl und Alkoxyalkyl.
- R¹: wird besonders bevorzugt ausgewählt aus der Gruppe, bestehend aus Methyl, Ethyl, Propyl, Vinyl, Allyl, Propargyl, Cyclopropyl, Alkoxyalkyl, 2-Fluor-ethyl, 2,2-Difluor-ethyl und 2-Fluor-cyclopropyl.
- R¹: wird ganz besonders bevorzugt ausgewählt aus der Gruppe, bestehend aus Methyl, Ethyl, n-Propyl, n-Prop-2-enyl, n-Prop-2-inyl, Cyclopropyl, Methoxyethyl, 2-Fluorethyl und 2,2-Difluor-ethyl.
- R²: wird bevorzugt ausgewählt aus der Gruppe, bestehend aus C₁-C₁₂-Alkyl, C₆-Aryl und Aralkyl.
- R²: wird besonders bevorzugt ausgewählt aus der Gruppe, bestehend aus C₁-C₁₂-Alk-yl.
- R²: wird ganz besonders bevorzugt ausgewählt aus der Gruppe, bestehend aus Methyl und Ethyl.

In einer bevorzugten Ausführungsform der vorliegenden Erfindung werden in dem erfindungsgemäßen Verfahren Ausgangsverbindungen der allgemeinen Formeln (II) und (III) eingesetzt, in welchen die Substituenten A, Z, R¹ und R² die vorstehend erwähnten bevorzugten Bedeutungen aufweisen.

In einer besonders bevorzugten Ausführungsform der vorliegenden Erfindung werden in dem erfindungsgemäßen Verfahren Ausgangsverbindungen der allgemeinen Formeln (II) und (III) eingesetzt, in welchen die Substituenten A, Z, R¹ und R² die vorstehend erwähnten besonders bevorzugten Bedeutungen aufweisen.

In einer ganz besonders bevorzugten Ausführungsform der vorliegenden Erfindung werden in dem erfindungsgemäßen Verfahren Ausgangsverbindungen der allgemeinen Formeln (II) und (III) eingesetzt, in welchen die Substituenten A, Z, R¹ und R² die vorstehend erwähnten ganz besonders bevorzugten Bedeutungen aufweisen.

Im Rahmen der vorliegenden Erfindung wird - unabhängig von den einzelnen oben erwähnten bevorzugten, besonders bevorzugten und ganz besonders bevorzugten Bedeutungen - den einzelnen verwendeten Resten im Allgemeinen folgende Bedeutung zugewiesen:
Unter dem Begriff "Alkyl", entweder in Alleinstellung oder aber in Kombination mit weiteren Begriffen, wie beispielsweise Halogenalkyl, Alkoxyalkyl, Cycloalkylalkyl, Halogencycloalkylalkyl und Arylalkyl, wird im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, isoPropyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste, speziell Methyl und Ethyl.

Unter dem Begriff "Alkenyl" wird erfindungsgemäß ein linearer oder verzweigter C₁-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind C₂-C₆-Alkenylreste und besonders bevorzugt sind C₂-C₄-Alkenylreste.

Unter dem Begriff "Alkinyl" wird erfindungsgemäß ein linearer oder verzweigter C₃-C₁₂-Alkinylrest, welcher mindestens eine Dreifachbindung aufweist, beispielsweise Ethinyl, 1-Propinyl und Propargyl, verstanden. Bevorzugt hiervon sind C₃-C₆-Alkinylreste und besonders bevorzugt sind C₃-C₄-Alkinylreste. Der Alkinylrest kann dabei auch mindestens eine Doppelbindung aufweisen.

Unter dem Begriff "Cycloalkyl" wird erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃-C₆-Cycloalkylreste.

Unter dem Begriff "Aryl" wird erfindungsgemäß ein aromatischer Rest mit 6 bis 14 Kohlenstoffatomen, vorzugsweise Phenyl, verstanden.

Unter dem Begriff "Arylalkyl" wird eine Kombination von erfindungsgemäß definierten Resten "Aryl" und "Alkyl" verstanden, wobei der Rest im Allgemeinen über die Alkylgrupe gebunden wird, Beispiele hierfür sind Benzyl, Phenylethyl oder α-Methylbenzyl, wobei Benzyl besonders bevorzugt ist.

Im Rahmen der vorliegenden Erfindung werden durch Halogen substituierte Reste, beispielsweise Halogenalkyl, einfach oder mehrfach bis zur maximal möglichen Substituentenzahl halogenierte Reste verstanden. Bei mehrfacher Halogenierung können die Halogenatome gleich oder verschieden sein. Halogen steht dabei für Fluor, Chlor, Brom oder Iod, insbesondere für Fluor, Chlor oder Brom.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Gegebenenfalls substituierte Reste können einfach oder mehrfach substituiert sein, wobei bei einer Mehrfachsubstitution die Substituenten gleich oder verschieden sein können.

Die Verbindungen der allgemeinen Formel (II) können in unterschiedlichen tautomeren Formen vorliegen:

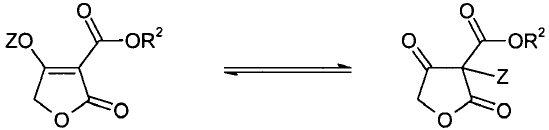

Insbesondere wenn Z Wasserstoff darstellt, liegen die Verbindungen aufgrund der Keto-Enol-Tautomerie in unterschiedlichen Formen vor. Im Rahmen der vorliegenden Erfindung sind - unabhängig von der Darstellungsweise der Verbindung der allgemeinen Formel (II) - alle tautomeren Strukturen der allgemeinen Formel (II) umfasst.

Die Verbindungen der allgemeinen Formel (II) sind zumindest partiell aus dem Stand der Technik bekannt. So sind beispielsweise folgende Verbindungen der allgemeinen Formel (II) mit Z gleich Wasserstoff oder Kalium und R² gleich Ethyl sowie die Herstellung dieser Verbindungen in J. Chem. Soc., Perkin Trans. 1, 1985, Seiten 1567 bis 1576 beschrieben:

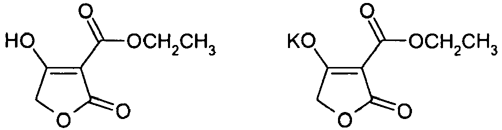

Die Synthese entsprechender abgewandelter Derivate der Verbindungen der allgemeinen Formel (II) kann gemäß nachfolgendem Schema 3 beispielsweise ausgehend von Kaliumsalzen der Malonsäureester der allgemeinen Formel (IV) über die Verbindung der allgemeinen Formel (V) unter Verwendung von Alkoholatbasen, wie Natriummethylat NaOCH₃ mit R² = CH₃ und Z = Na, durchgeführt werden:

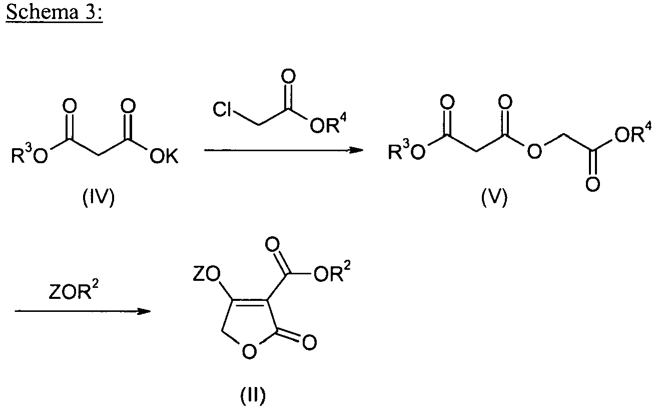

In Abhängigkeit der sterischen Größe des Alkoholatrestes kann es, wie im Fall des Natriumalkoholats im Schema 3 gezeigt ist, zu einem Austausch des Esterrestes kommen (in Schema 3 wird der Esterrest in der Verbindung der allgemeinen Formel (V) gegen R² ausgetauscht). Wird jedoch ein sterisch anspruchsvoller Rest ausgewählt, so kann der Esteraustausch reduziert oder sogar unterdrückt werden.

Wird zum Beispiel in dieser Synthese Kalium-tert.-butylat als Base verwendet, so erfolgt kein Austausch des Substituenten R³, welcher damit in der Zielverbindung verbleibt (Schema 4).

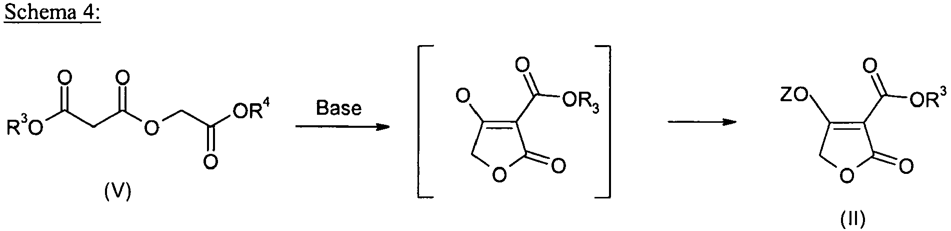

Darüber hinaus werden die Verbindungen der allgemeinen Formel (II) mit Z = Wasserstoff ausgehend von Malonester und Chloracetylchlorid nach folgendem Schema 5 gemäß dem Stand der Technik Berichte der Deutschen Chemischen Gesellschaft (1911), 44, 1759 - 1765 hergestellt (in der Verbindung der allgemeinen Form (II) ist die Ketoform dargestellt, d.h. Z ist gleich Wasserstoff):

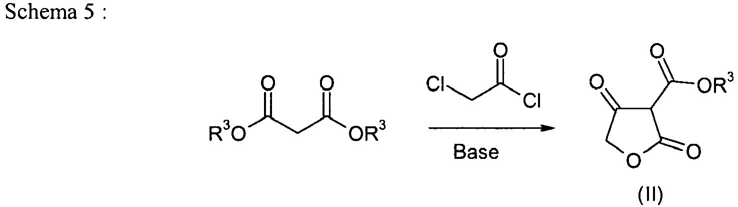

Hierbei weisen die einzelnen Reste, falls vorhanden, im Allgemeinen folgende Bedeutung auf:
- R²: wie oben stehend definiert;
- R³: Alkyl, mit Ausnahme von Ethyl, Cycloalkyl, Halogenalkyl, Aryl oder Arylalkyl;
- R⁴: Alkyl, Cycloalkyl, Halogenalkyl, Aryl oder Arylalkyl; und
- Z: wie oben stehend definiert.

Die als Edukte verwendeten Kaliumsalze der Malonsäureester der allgemeinen Formel (IV) sind kommerziell erhältlich oder können nach aus dem Stand der Technik bekannten Verfahren hergestellt werden (vgl. J. Am. Chem. Soc., 1944, Nr. 66, Seite 1286).

Aus dem Stand der Technik sind weder die Natriumsalze (Z = Na) noch entsprechende Methylester (R² = CH₃) der allgemeinen Formel (II) bekannt, welche daher als Intermediate der erfindungsgemäßen Synthese - wie weiter unten beschrieben - aber nicht einen Gegenstand der vorliegenden Erfindung bilden.

Insbesondere die Verwendung der entsprechenden Natriumsalze der allgemeinen Formel (II) (X = Na) ist in dem erfindungsgemäßen Verfahren bevorzugt, da die Natriumsalze - im Gegensatz zu den aus dem Stand der Technik bekannten Kaliumsalzen - aufgrund des Austausches der Base Kalium-tert.-butylat durch Natriummethylat kostengünstig synthetisiert werden können.

Weiterer Gegenstand der vorliegenden Beschreibung ist darüber hinaus - wie ebenfalls weiter unten beschrieben - das oben in Schema 3 beschriebene Verfahren zur Herstellung der entsprechenden Natriumsalze (Z = Na) und/oder Methylester (R² = CH₃) der allgemeinen Formel (II) gemäß dem oben dargestellten Schema.

Die für die erfindungsgemäße Umsetzung erforderlichen Amine der allgemeinen Formel (III) sind kommerziell erhältlich oder können nach literaturbekannten Verfahren hergestellt werden (vgl. z.B. S. Patai " The Chemistry of Amino Group", Interscience Publishers, New York, 1968).

Die Umsetzung der Esterverbindungen der allgemeinen Formel (II) mit den Aminen der allgemeinen Formel (III) kann in Gegenwart von Lösungsmitteln (Verdünnungsmitteln) durchgeführt werden. Lösungsmittel werden vorteilhaft in einer solchen Menge eingesetzt, dass das Reaktionsgemisch während des ganzen Verfahrens gut rührbar bleibt. Als Lösungsmittel zur Durchführung des erfindungsgemäßen Verfahrens kommen alle unter den Reaktionsbedingungen inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Ether, wie Ethylpropylether, Methyl-tert-butylether, *n*-Butylether, Anisol, Phenetol, Cyclohexylmethylether, Dimethylether, Diethylether, Dimethylglycol, Diphenylether, Dipropylether, Diisopropylether, Di-*n*-butylether, Diisobutylether, Diisoamylether, Ethylenglycoldimethylether, Isopropylethylether, Methyl-tert-butylether, Tetrahydrofuran, Methyl-Tetrahydrofuran, Dioxan, Dichlordiethylether; Methyl-THF und Polyether des Ethylenoxids und/oder Propylenoxids; Nitrokohlenwasserstoffe wie Nitromethan, Nitroethan, Nitropropan, Nitrobenzol, Chlornitrobenzol, o-Nitrotoluol; Nitrile wie Acetonitril, Methylnitril, Propionitril, Butyronitril, Isobutyronitril, Benzonitril, Phenylnitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, n-Hexan, n-Heptan, n-Oktan, Nonan beispielsweise sogenannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, N,N-Dimethyl-acetamid, *N*-Methyl-formamid, *N,N*-Dimethyl-formamid, *N,N-*Dipropyl-formamid, *N,N-*Dibutyl-formamid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N*-Formyl-piperidin, *N,N*'-1,4-Diformyl-piperazin; und aliphatische Alkohole, wie Methanol, Ethanol, n-Propanol und iso-Propanol und n-Butanol.

Die erfindungsgemäße Umsetzung wird vorzugsweise in einem Lösungsmittel durchgeführt, welches ausgewählt ist aus der Gruppe, bestehend aus Dioxan, Butyronitril, Propionitril, Acetonitril, DME, Toluol, Methyl-THF, Dichlorbenzol, Chlorbenzol, n-Heptan, iso-Butanol, n-Butanol, Ethanol, Methyl-tert.-butylether, Isopropylethylether und Mischungen davon.

Gegebenenfalls ist es in Abhängigkeit der exakten Ausgangsverbindungen auch möglich, die Umsetzung in Substanz, d.h. ohne Zusatz an Lösungsmitteln, durchzuführen.

Die Umsetzung der Verbindungen der allgemeinen Formel (II) mit den Aminen der allgemeinen Formel (III) wird vorzugsweise in Gegenwart einer Brønsted-Säure durchgeführt.

Dabei ist es möglich, sowohl organische als auch anorganische Säuren zu verwenden. Vorzugsweise werden anorganische Säuren, beispielsweise Phosphorsäure (H₃PO₄), Schwefelsäure (H₂SO₄), Salzsäure (HCl), Bromwasserstoffsäure (HBr), Flusssäure (HF) oder Kaliumhydrogensulfat (KHSO₄), verwendet. Die einzelnen Säuren können dabei sowohl in wasserfreier Form als auch in wasserhaltiger Form, beispielsweise als 85%ige Phosphorsäure oder 37%ige Salzsäure, d.h. insbesondere in Formen, in welchen die Säuren kommerziell erhältlich sind, verwendet werden. Beispiele für geeignete organische Säuren sind Trifluoressigsäure, Essigsäure, Methansulfonsäure und p-Toluolsulfonsäure. Von den zuvor genannten Säuren sind insbesondere Phosphorsäure, Schwefelsäure, Kaliumhydrogensulfat und Trifluoressigsäure bevorzugt.

Die Umsetzung zur Herstellung der Verbindungen der allgemeinen Formel (I) kann im Allgemeinen im Vakuum, bei Normaldruck oder unter Überdruck durchgeführt werden. Die angewendeten Temperaturen können ebenfalls, in Abhängigkeit der verwendeten Substrate, variieren und sind für den Fachmann durch Routineversuche leicht zu ermitteln. Beispielsweise kann die Umsetzung zur Herstellung der Verbindungen der allgemeinen Formel (I) bei einer Temperatur von 20 bis 200 °C, vorzugsweise 20 bis 150 °C, durchgeführt werden. Besonders bevorzugt wird die Umsetzung bei Temperaturen von 20 bis 150 °C durchgeführt werden.

Die Stöchiometrie der eingesetzten Ausgangsverbindungen der allgemeinen Formel (II) und (III) kann in weiten Bereichen variieren und unterliegt im Allgemeinen keiner besonderen Beschränkung. Geeignete Stöchiometrien der eingesetzten Ausgangsverbindungen der allgemeinen Formel (II) und (III) können von dem Fachmann leicht durch Routineversuche ermittelt werden. So kann das molare Verhältnis der Verbindung der allgemeinen Formel (II) zu dem eingesetzten Amin der allgemeinen Formel (III) beispielsweise 0,5 bis 10, insbesondere 1 bis 6, speziell 1,05 bis 2, betragen. Der Einsatz größerer Mengen an Verbindung der allgemeinen Formel (III) ist grundsätzlich möglich, führt jedoch zu keiner bevorzugten Ausführungsform und ist aus wirtschaftlichen Gründen nachteilig.

Zum Ende der Reaktion kann das Reaktionswasser durch Destillation eines Teils des Lösungsmittel als Azeotrop entfernt werden. Bei hochsiedenden Lösungsmittel kann dies im Vakuum erfolgen. Durch diesen Vorgang erreicht man im Allgemeinen einen quantitativen Umsatz.

Falls die Umsetzung in einem Lösungsmittel durchgeführt wird, kann das Lösungsmittel nach dem Reaktionsende durch Abdestillieren entfernt werden. Dieses kann unter Normaldruck oder erniedrigtem Druck bei Raumtemperatur oder erhöhten Temperaturen erfolgen.

Die Isolierung der gewünschten Verbindungen der allgemeinen Formel (I) kann beispielsweise auch durch Kristallisation erfolgen.

Wie bereits erwähnt sind die Verbindungen der allgemeinen Formel (II) teilweise nicht aus dem Stand der Technik bekannt.

Beschrieben werden ferner Verbindungen der allgemeinen Formel (IIa) 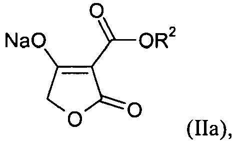 in welchen
- R²: wie oben stehend definiert ist.

Die Verbindungen der allgemeinen Formel (IIa) liegen als Natriumsalze vor.

Beschrieben werden ferner Verbindungen der allgemeinen Formel (IIb) 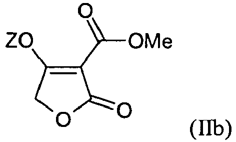 in welchen ausgewählt ist aus der Gruppe, bestehend aus Alkalimetallen und Erdalkalimetallen.

Die Verbindungen der allgemeinen Formel (II), insbesondere die Verbindungen der Formel (IIa) und (IIb), werden ausgehend von den Verbindungen der allgemeinen Formel (IV) durch Cyclisierung erhalten.

Für die Cyclisierung selbst kann jede beliebige Base wie beispielsweise Kalium-tert.-butylat oder Natriummethylat verwendet werden, wobei aus ökonomischen Gründen Natriummethylat bevorzugt ist.

Geeignete Lösungsmittel für die Cyclisierungsumsetzung sind beispielsweise diejenigen, welche auch für die erfindungsgemäße Herstellung der 4-Amino-but-2-enolid-Verbindungen der allgemeinen Formel (I) verwendet werden.

Die Cyclisierung kann im Allgemeinen im Vakuum, bei Normaldruck oder unter Überdruck durchgeführt werden. Die angewendeten Temperaturen können ebenfalls, in Abhängigkeit der verwendeten Substrate, variieren und sind für den Fachmann durch Routineversuche leicht zu ermitteln. Beispielsweise kann die Umsetzung zur Herstellung der Verbindungen der allgemeinen Formel (V) bei einer Temperatur von 20 bis 200 °C, vorzugsweise 20 bis 150 °C, durchgeführt werden. Besonders bevorzugt wird die Umsetzung bei Normaldruck und Temperaturen von 20 bis 150 °C durchgeführt werden.

Die Stöchiometrie der eingesetzten Ausgangsverbindungen der allgemeinen Formel (V) zu der verwendeten Base kann in weiten Bereichen variieren und unterliegt im Allgemeinen keiner besonderen Beschränkung. Geeignete Stöchiometrien der eingesetzten Ausgangsverbindungen der allgemeinen Formel (V) und der Base können von dem Fachmann leicht durch Routineversuche ermittelt werden. So kann das molare Verhältnis der Verbindung der allgemeinen Formel (V) zu der Base beispielsweise 0,9 bis 10, insbesondere 1 bis 5, speziell 1 bis 2, betragen.

Die Verbindungen der allgemeinen Formel (V) können nun wiederum durch Umsetzung der bekannten bzw. kommerziell erhältlichen Kaliumsalze der Malonsäureester mit Halogenessigsäurealkylestern hergestellt werden. Hierbei können im Allgemeinen Bromessigsäurealkylester, insbesondere Bromessigsäureethylester, und Chloressigsäurealkylester, insbesondere Chloressigsäureethylester, verwendet werden. Dabei sind die entsprechenden Chlorderivate besonders bevorzugt, da die korrespondierenden Bromderivate teuerer sind.

Die vorliegende Beschreibung betrifft daher auch die Herstellung der Verbindungen der allgemeinen Formel (V) 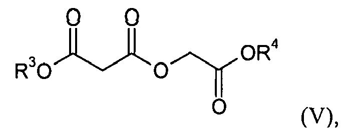 dadurch gekennzeichnet, dass man Verbindungen der allgemeinen Formel (IV) 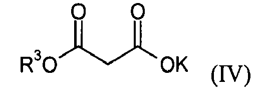 mit Chloressigsäurealkylestern, insbesondere Chloressigsäureethylester, der allgemeinen Formel (VI) 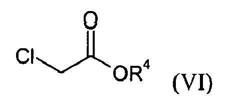 umsetzt, wobei
die einzelnen Substituenten R³ und R⁴ die oben stehende Bedeutung aufweisen.

Diese Umsetzungen zu den Verbindungen der allgemeinen Formel (V) werden üblicherweise in einem Lösungsmittel durchgeführt.

Geeignete Lösungsmittel sind beispielsweise diejenigen, welche auch für die erfindungsgemäße Herstellung der 4-Amino-but-2-enolid-Verbindungen der allgemeinen Formel (I) verwendet werden. Hiervon besonders bevorzugt ist Dimethylformamid und Dimethylacetamid.

Die Umsetzung zur Herstellung der Verbindungen der allgemeinen Formel (V) kann im Allgemeinen im Vakuum, bei Normaldruck oder unter Überdruck durchgeführt werden. Die angewendeten Temperaturen können ebenfalls, in Abhängigkeit der verwendeten Substrate, variieren und sind für den Fachmann durch Routineversuche leicht zu ermitteln. Beispielsweise kann die Umsetzung zur Herstellung der Verbindungen der allgemeinen Formel (V) bei einer Temperatur von 20 bis 200 °C, vorzugsweise 20 bis 150 °C, durchgeführt werden. Besonders bevorzugt wird die Umsetzung bei Normaldruck und Temperaturen von 20 bis 150 °C durchgeführt werden.

Die Stöchiometrie der eingesetzten Ausgangsverbindungen der allgemeinen Formel (IV) und (VI) kann in weiten Bereichen variieren und unterliegt im Allgemeinen keiner besonderen Beschränkung. Geeignete Stöchiometrien der eingesetzten Ausgangsverbindungen der allgemeinen Formel (IV) und (VI) können von dem Fachmann leicht durch Routineversuche ermittelt werden. So kann das molare Verhältnis der Verbindung der allgemeinen Formel (IV) zu dem eingesetzten Ester der allgemeinen Formel (VI) beispielsweise 5 bis 0,8, insbesondere 3 bis 0,9, speziell 2 bis 1, betragen.

Falls die Umsetzung in einem Lösungsmittel durchgeführt wird, kann das Lösungsmittel nach dem Reaktionsende durch Abdestillieren entfernt werden. Dieses kann unter Normaldruck oder erniedrigtem Druck bei Raumtemperatur oder erhöhten Temperaturen erfolgen.

Die Isolierung der gewünschten Verbindungen der allgemeinen Formel (V) kann beispielsweise auch durch Kristallisation erfolgen.

Darüber hinaus ist es auch möglich, die Verbindungen der allgemeinen Formel (V) unmittelbar, d.h. insbesondere ohne Aufreinigung, zu den Verbindungen der allgemeinen Formel (II) umzusetzen.

Die vorliegende Erfindung wird anhand der nachfolgenden Beispiele näher erläutert, wobei die Beispiele nicht in die Erfindung einschränkende Weise zu interpretieren sind (Nur vom Anspruchsumfang umfasste Verfahren sind erfindungsgemäss).

### Herstellungsbeispiele:

### Beispiel 1

Zu einer Suspension von 10,4 g 4-(Ethoxycarbonyl)-5-oxo-2,5-dihydrofuran-3-olat-Kaliumsalz und 7,5 g N-[(6-Chlorpyridin-3-yl)methyl]-2,2-difluorethylamin in 68 ml Butyronitril werden bei Raumtemperatur 11,5 g Kaliumhydrogensulfat zugesetzt. Die Mischung wird bei einer Temperatur von 90 bis 95 °C 3 Stunden gerührt. Anschließend wird auf Raumtemperatur abgekühlt, 120 ml Wasser und 120 ml Dichlormethan zugegeben, die organische Phase abgetrennt und die wässrige Phase wird zweimal mit je 120 ml Methylenchlorid extrahiert. Die vereinigten organischen Phasen werden zur Trockene eingeengt. Man erhält 11,4 g 4-[[(6-Chlorpyridin-3-yl)methyl)(2,2-difluorethyl)amino]furan-2(5H)-on in einer Reinheit von 84 % (dies entspricht 92 % Ausbeute).

### Beispiel 2

Zu einer Suspension von 5 g 4-(Methoxycarbonyl)-5-oxo-2,5-dihydrofuran-3-olat-Natriumsalz und 4,2 g N-[(6-Chlorpyridin-3-yl)methyl]-2,2-difluorethylamin in 35 ml Butyronitril werden bei Raumtemperatur 7,5 g Kaliumhydrogensulfat zugesetzt. Die Mischung wird bei einer Temperatur von 90 °C 3 Stunden gerührt. Anschließend wird auf Raumtemperatur abgekühlt, mit 60 ml Wasser und 60 ml Dichlormethan versetzt. Die organische Phase wird abgetrennt und die wässrige Phase wird zweimal mit je 30 ml Methylenchlorid extrahiert. Die vereinigten organischen Phasen werden zur Trockene eingeengt. Man erhält 5,95 g 4-[[(6-Chlorpyridin-3-yl)methyl](2,2-difluorethyl)amino]furan-2(5H)-on in einer Reinheit von 89 % (dies entspricht 91 % Ausbeute).

### Beispiel 3

Zu einer Suspension von 10 g 4-(Methoxycarbonyl)-5-oxo-2,5-dihydrofuran-3-olat-Natriumsalz und 4-(Ethoxycarbonyl)-5-oxo-2,5-dihydrofuran-3-olat-Natriumsalz (Verhältnis 77 : 23) und 3,5 g N-[(6-Chlorpyridin-3-yl)methyl]-methylamin in 35 ml Butyronitril werden bei Raumtemperatur 7,5 g Kaliumhydrogensulfat zugesetzt. Die Mischung wird bei einer Temperatur von 90 °C 3 Stunden gerührt. Anschließend wird auf Raumtemperatur abgekühlt und das Lösungsmittel komplett entfernt. Der Rückstand wird mit 50 ml Wasser versetzt und zweimal mit je 30 ml Dichlormethan extrahiert. Die vereinigten organischen Phasen werden zur Trockene eingeengt. Man erhält 4,7 g 4-[[(6-Chlorpyridin-3-yl)methyl]methylamino]furan-2(5H)-on in einer Reinheit von 95 % (dies entspricht 82 % Ausbeute).

### Beispiel 4

Zu einer Suspension von 78,9 g Kaliummonoethylmalonat in 500 ml Dimethylformamid werden bei 35 °C 55 g Chloressigsäuremethylester zugetropft und 8 Stunden bei 35 °C gerührt. Das Lösungsmittel wird im Vakuum entfernt und der Rückstand mit 100 ml Wasser und 100 ml Toluol versetzt. Die Phasen werden getrennt und die wässrige Phase wird mit 100 ml Toluol gewaschen. Die vereinigten organischen Phasen werden über Magnesiumsulfat getrocknet und im Vakuum eingeengt. Man erhält 77,6 g 2-Methoxy-2-oxoethylpropandicarbonsäuremethylester in einer Reinheit von 98 % (dies entspricht 80 % Ausbeute).
¹H-NMR (CDCl₃, 298K) δ: 3,51 s (2H), 3,77 s (3H), 3,78 s (3H), 4,69 s (2H)

### Beispiel 5

Zu 19,6 g 2-Methoxy-2-oxoethylpropandicarbonsäuremethylester werden bei 40 °C 18 g einer 30 %igen Natriummethylat in Methanol zugetropft und 3 Stunden unter Rückfluss erhitzt. Anschließend wird auf Raumtemperatur abgekühlt, der Feststoff abgesaugt und mit 20 ml Methanol gewaschen. Das Produkt wird bei 50 °C im Vakuum getrocknet. Man erhält 15,6 g 4-(Methoxycarbonyl)-5-oxo-2,5-dihydrofuran-3-olat Natriumsalz in einer Reinheit von 99,9 % (dies entspricht 87 % Ausbeute).
¹H-NMR (D₂O, 298K) δ: 3,73 s (3H), 4,42 s (2H)

### Beispiel 6

Zu 23,1 g 2-Ethoxy-2-oxoethylpropandicarbonsäureethylester werden bei 40 °C 18 g einer 30 %igen Natriummethylat in Methanol zugetropft und 2 Stunden unter Rückfluss erhitzt. Anschließend wird auf 0 °C abgekühlt, der Feststoff abgesaugt und mit 10 ml Methanol gewaschen. Das Produkt wird bei 50 °C im Vakuum getrocknet. Man erhält eine Mischung von 15,7 g 4-(Methoxycarbonyl)-5-oxo-2,5-dihydrofuran-3-olat-Natriumsalz und 4-(Ethoxycarbonyl)-5-oxo-2,5-dihydrofuran-3-olat-Natriumsalz (Verhältnis 77 : 23) (dies entspricht 86 % Ausbeute).

### Beispiel 7

Zu einer Suspension von 5,2 g 4-(Methoxycarbonyl)-5-oxo-2,5-dihydrofuran-3-ol und 5 g N-[(6-Chlorpyridin-3-yl)methyl]-2,2-difluorethylamin in 70 ml Butyronitril werden bei Raumtemperatur 4,5 g Kaliumhydrogensulfat zugesetzt. Die Mischung wird bei einer Temperatur von 120°C 3 Stunden gerührt. Anschließend wird auf Raumtemperatur abgekühlt, mit 60 ml Wasser und 60 ml Dichlormethan versetzt. Die organische Phase wird abgetrennt und die wässrige Phase wird zweimal mit je 30 ml Methylenchlorid extrahiert. Die vereinigten organischen Phasen werden zur Trockene eingeengt. Man erhält 7,6 g 4-[[(6-Chlorpyridin-3-yl)methyl](2,2-difluorethyl)amino]furan-2(5H)-on in einer Reinheit von 85 % (dies entspricht 94 % Ausbeute).

### Beispiel 8

Zu einer Suspension von 18 g 4-(Methoxycarbonyl)-5-oxo-2,5-dihydrofuran-3-ol und 15 g N-[(6-Chlorpyridin-3-yl)methyl]-methylamin in 210 ml Butyronitril werden bei Raumtemperatur 16 g Kaliumhydrogensulfat zugesetzt. Die Mischung wird bei einer Temperatur von 115°C 5 Stunden gerührt. Anschließend wird auf Raumtemperatur abgekühlt, mit 150 ml Wasser und 70 ml Dichlormethan versetzt. Die organische Phase wird abgetrennt und die wässrige Phase wird zweimal mit je 70 ml Methylenchlorid extrahiert. Die vereinigten organischen Phasen werden zur Trockene eingeengt. Man erhält 24 g 4-[[(6-Chlorpyridin-3-yl)methyl](methyl)amino]furan-2(5H)-on in einer Reinheit von 88 % (dies entspricht 92 % Ausbeute).

## Patentansprüche

1. Verfahren zur Herstellung von 4-Aminobut-2-enolidverbindungen der allgemeinen Formel (I): 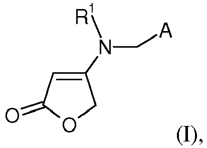 **dadurch gekennzeichnet, dass** man Verbindungen der allgemeinen Formel (II) 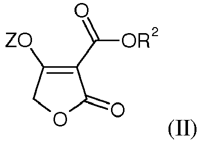 mit Aminen der allgemeinen Formel (III) 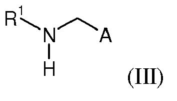 umsetzt, wobei die einzelnen Reste folgende Bedeutung aufweisen:
R¹ Wasserstoff, Alkyl, Halogenalkyl, Alkenyl, Halogenalkenyl, Alkinyl, Cycloalkyl, Cycloalkylalkyl, Halogencycloalkyl, Alkoxy, Alkyoxyalkyl, Halogencyclo-alkylalkyl oder Arylalkyl;
R² Alkyl, Aryl oder Arylalkyl;
Z Wasserstoff, Alkalimetall oder Erdalkalimetall;
A Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder 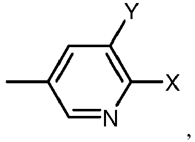
in welchem
X für Halogen, Alkyl oder Halogenalkyl steht und
Y für Halogen, Alkyl, Halogenalkyl, Halogenalkoxy, Azido oder Cyan steht.

2. Verfahren nach Anspruch 1, **dadurch gekennzeichnet, dass** die Verbindungen der allgemeinen Formel (II) durch Umsetzung von Verbindungen der allgemeinen Formel (V) 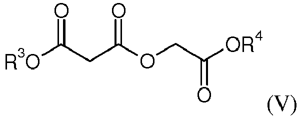 mit Basen, insbesondere Alkoholatbasen, erhalten werden.

3. Verfahren nach Anspruch 2, **dadurch gekennzeichnet, dass** die Verbindungen der allgemeinen Formel (II) gemäß nachfolgendem Schema ausgehend von Verbindungen der allgemeinen Formel (V) durch Umsetzung mit Natriumalkoholat erhalten werden: 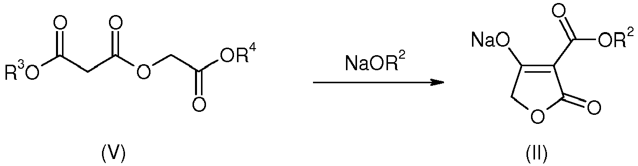 wobei die einzelnen Reste im Allgemeinen folgende Bedeutung aufweisen:
R² wie im Anspruch 1 definiert;
R³ Alkyl, mit Ausnahme von Ethyl, Cycloalkyl, Halogenalkyl, Aryl oder Arylalkyl; und
R⁴ Alkyl, Cycloalkyl, Halogenalkyl, Aryl oder Arylalkyl.

4. Verfahren nach Anspruch 2, **dadurch gekennzeichnet, dass** die Verbindungen der allgemeinen Formel (II) gemäß nachfolgendem Schema ausgehend von Verbindungen der allgemeinen Formel (V) durch Umsetzung mit Kalium-tert.-butylat erhalten werden: 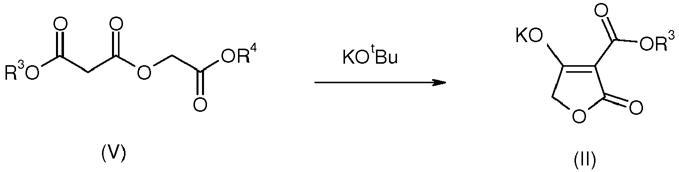 wobei die einzelnen Reste im Allgemeinen folgende Bedeutung aufweisen:
R³ Alkyl, mit Ausnahme von Ethyl, Cycloalkyl, Halogenalkyl, Aryl oder Arylalkyl; und
R⁴ Alkyl, Cycloalkyl, Halogenalkyl, Aryl oder Arylalkyl.

5. Verfahren nach Anspruch 1, **dadurch gekennzeichnet, dass** die Verbindungen der allgemeinen Formel (II) gemäß nachfolgendem Schema ausgehend von Malonester und Chloracetylchlorid erhalten werden: 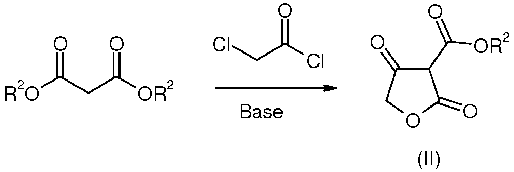 wobei der Rest R² die in Anspruch 1 definierte Bedeutung aufweist.

6. Verfahren nach einem der Ansprüche 2 bis 4, **dadurch gekennzeichnet, dass** man zunächst Verbindungen der allgemeinen Formel (V) 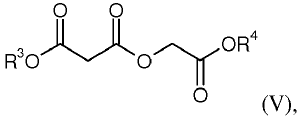 herstellt, indem man Verbindungen der allgemeinen Formel (IV) 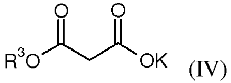 mit Chloressigsäurealkylestern, insbesondere Chloressigsäureethylester, der allgemeinen Formel (VI) 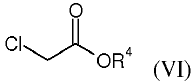 umsetzt, wobei
die Substituenten R³ und R⁴ die folgende Bedeutung aufweisen:
R³ Alkyl, mit Ausnahme von Ethyl, Cycloalkyl, Halogenalkyl, Aryl oder Arylalkyl;
R⁴ Alkyl, Cycloalkyl, Halogenalkyl, Aryl oder Arylalkyl,
und dann
die Verbindungen der allgemeinen Formel (V) in dem Verfahren gemäß einem der Ansprüche 2 bis 4 als Ausgangsverbindung verwendet.

## Claims

1. Process for preparing 4-aminobut-2-enolide compounds of the general formula (I): 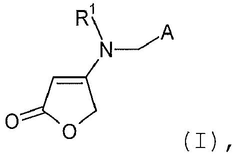 **characterized in that** compounds of the general formula (II) 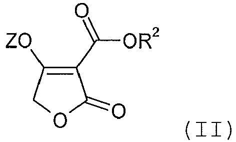 are reacted with amines of the general formula (III) 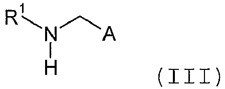 where the individual radicals are defined as follows:
R¹ is hydrogen, alkyl, haloalkyl, alkenyl, haloalkenyl, alkynyl, cycloalkyl, cycloalkylalkyl, halocycloalkyl, alkoxy, alkyoxyalkyl, halocycloalkylalkyl or arylalkyl;
R² is alkyl, aryl or arylalkyl;
Z is hydrogen, an alkali metal or an alkaline earth metal;
A is pyrid-2-yl or pyrid-4-yl, or is pyrid-3-yl which is optionally 6-substituted by fluorine, chlorine, bromine, methyl, trifluoromethyl or trifluoromethoxy, or is pyridazin-3-yl which is optionally 6-substituted by chlorine or methyl, or is pyrazin-3-yl or is 2-chloropyrazin-5-yl or is 1,3-thiazol-5-yl which is optionally 2-substituted by chlorine or methyl, or
pyrimidinyl, pyrazolyl, thiophenyl, oxazolyl, isoxazolyl, 1,2,4-oxadiazolyl, isothiazolyl, 1,2,4-triazolyl or 1,2,5-thiadiazolyl, which is optionally substituted by fluorine, chlorine, bromine, cyano, nitro, C₁-C₄-alkyl (which is optionally substituted by fluorine and/or chlorine), C₁-C₃-alkylthio (which is optionally substituted by fluorine and/or chlorine), or C₁-C₃-alkylsulphonyl (which is optionally substituted by fluorine and/or chlorine), or 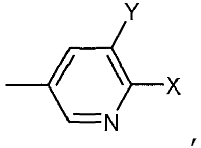 in which
X is halogen, alkyl or haloalkyl and
Y is halogen, alkyl, haloalkyl, haloalkoxy, azido or cyano.

2. Process according to Claim 1, **characterized in that** the compounds of the general formula (II) are obtained by reacting compounds of the general formula (V) 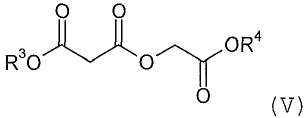 with bases, especially alkoxide bases.

3. Process according to Claim 2, **characterized in that** the compounds of the general formula (II) are obtained by the following scheme proceeding from compounds of the general formula (V) by reaction with sodium alkoxide: 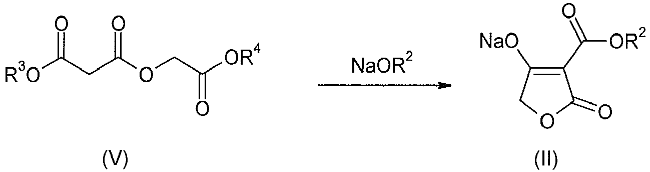 where the individual radicals are generally defined as follows:
R² is as defined in Claim 1;
R³ is alkyl, with the exception of ethyl, cycloalkyl, haloalkyl, aryl or arylalkyl; and
R⁴ is alkyl, cycloalkyl, haloalkyl, aryl or arylalkyl.

4. Process according to Claim 2, **characterized in that** the compounds of the general formula (II) are obtained by the following scheme proceeding from compounds of the general formula (V) by reaction with potassium tert-butoxide: 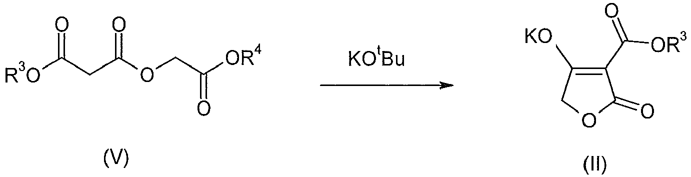 where the individual radicals are generally defined as follows:
R³ is alkyl, with the exception of ethyl, cycloalkyl, haloalkyl, aryl or arylalkyl; and
R⁴ is alkyl, cycloalkyl, haloalkyl, aryl or arylalkyl.

5. Process according to Claim 1, **characterized in that** the compounds of the general formula (II) are obtained by the following scheme proceeding from malonic esters and chloroacetyl chloride: 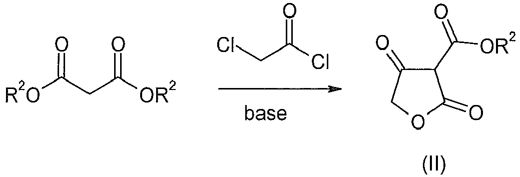 where the R² radical is as defined in Claim 1.

6. Process according to one of Claims 2 to 4, **characterized in that** first compounds of the general formula (V) 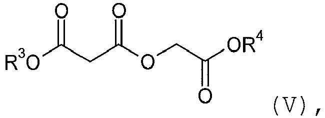 are prepared by reacting compounds of the general formula (IV) 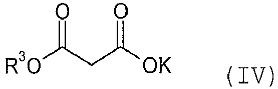 with alkyl chloroacetates, especially ethyl chloroacetate, of the general formula (VI) 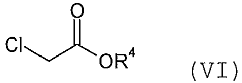 where
the substituents R³ and R⁴ are defined as follows:
R³ is alkyl, with the exception of ethyl, cycloalkyl, haloalkyl, aryl or arylalkyl;
R⁴ is alkyl, cycloalkyl, haloalkyl, aryl or arylalkyl, and then
the compounds of the general formula (V) are used as the starting compound in the process according to one of Claims 2 to 4.

## Revendications

1. Procédé de fabrication de composés de 4-aminobut-2-énolide de formule générale (I) : 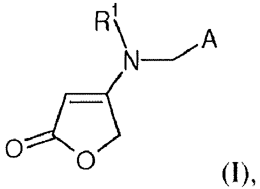 **caractérisé en ce que** des composés de formule générale (II) 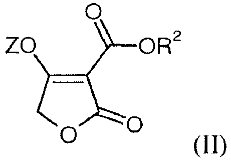 sont mis en réaction avec des amines de formule générale (III) 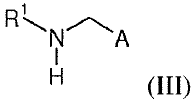 les radicaux individuels ayant la signification suivante :
R¹ hydrogène, alkyle, halogénoalkyle, alcényle, halogénoalcényle, alcynyle, cycloalkyle, cycloalkylalkyle, halogénocycloalkyle, alcoxy, alkyloxyalkyle, halogénocycloalkylalkyle ou arylalkyle ;
R² alkyle, aryle ou aryalkyle ;
Z hydrogène, métal alcalin ou métal alcalino-terreux ;
A pyrid-2-yle ou pyrid-4-yle ; ou pyrid-3-yle, qui est éventuellement substitué en position 6 par fluor, chlore, brome, méthyle, trifluorométhyle ou trifluorométhoxy ; ou pyridazin-3-yle, qui est éventuellement substitué en position 6 par chlore ou méthyle ; ou pyrazin-3-yle ou 2-chloro-pyrazin-5-yle ou 1,3-thiazol-5-yle, qui est éventuellement substitué en position 2 par chlore ou méthyle ; ou pyrimidinyle, pyrazolyle, thiophényle, oxazolyle, isoxazolyle, 1,2,4-oxadiazolyle, isothiazolyle, 1,2,4-triazolyle ou 1,2,5-thiadiazolyle, qui est éventuellement substitué par fluor, chlore, brome, cyano, nitro, alkyle en C₁-C₄ (qui est éventuellement substitué par fluor et/ou chlore), alkylthio en C₁-C₃ (qui est éventuellement substitué par fluor et/ou chlore) ou alkylsulfonyle en C₁-C₃ (qui est
éventuellement substitué par fluor et/ou chlore), ou 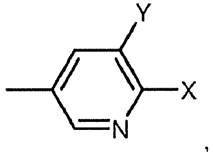
X représentant halogène, alkyle ou halogénoalkyle, et
Y représentant halogène, alkyle, halogénoalkyle, halogénoalcoxy, azido ou cyano.

2. Procédé selon la revendication 1, **caractérisé en ce que** les composés de formule générale (II) sont obtenus par mise en réaction de composés de formule générale (V) 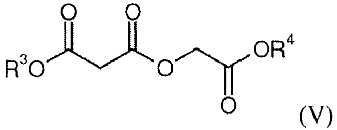 avec des bases, notamment des bases alcoolate.

3. Procédé selon la revendication 2, **caractérisé en ce que** les composés de formule générale (II) sont obtenus selon le schéma suivant à partir de composés de formule générale (V) par mise en réaction avec un alcoolate de sodium : 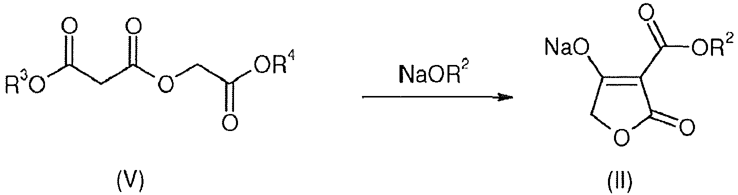 les radicaux individuels ayant généralement la signification suivante :
R² tel que défini dans la revendication 1 ;
R³ alkyle, à l'exception d'éthyle, cycloalkyle, halogénoalkyle, aryle ou arylalkyle ; et
R⁴ alkyle, cycloalkyle, halogénoalkyle, aryle ou arylalkyle.

4. Procédé selon la revendication 2, **caractérisé en ce que** les composés de formule générale (II) sont obtenus selon le schéma suivant à partir de composés de formule générale (V) par mise en réaction avec du tert.-butylate de potassium : 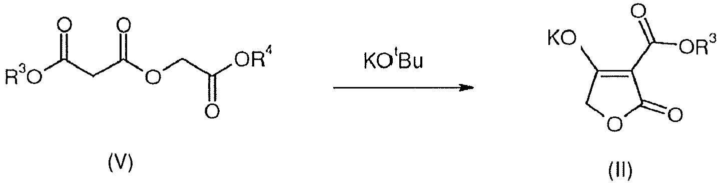 les radicaux individuels ayant généralement la signification suivante :
R³ alkyle, à l'exception d'éthyle, cycloalkyle, halogénoalkyle, aryle ou arylalkyle ; et
R⁴ alkyle, cycloalkyle, halogénoalkyle, aryle ou arylalkyle.

5. Procédé selon la revendication 1, **caractérisé en ce que** les composés de formule générale (II) sont obtenus selon le schéma suivant à partir d'ester malonique et de chlorure de chloroacétyle : 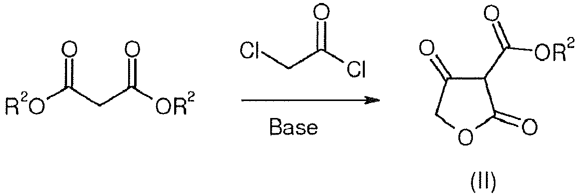 le radical R² ayant la signification définie dans la revendication 1.

6. Procédé selon l'une quelconque des revendications 2 à 4, **caractérisé en ce que**
des composés de formule générale (V) 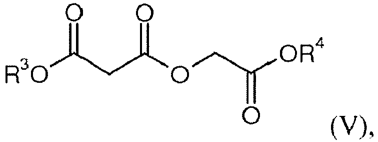 sont tout d'abord fabriqués, par mise en réaction de composés de formule générale (IV) 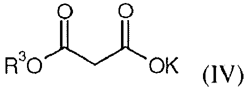 avec des esters alkyliques de l'acide chloroacétique, notamment l'ester éthylique de l'acide chloroacétique, de formule générale (VI) 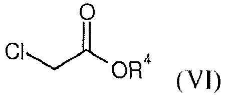 les substituants R³ et R⁴ ayant la signification suivante :
R³ alkyle, à l'exception d'éthyle, cycloalkyle, halogénoalkyle, aryle ou arylalkyle ; et
R⁴ alkyle, cycloalkyle, halogénoalkyle, aryle ou arylalkyle,
puis
les composés de formule générale (V) sont utilisés en tant que composé de départ dans le procédé selon l'une quelconque des revendications 2 à 4.
